# EUROPÄISCHE PATENTSCHRIFT

(11) **EP 1 295 600 B1**
(45) Veröffentlichungstag und Bekanntmachung des Hinweises auf die Patenterteilung: **05.04.2006**
(21) Anmeldenummer: 02021058.9
(22) Anmeldetag: 20.09.2002
(51) Int. Cl.: A61K 31/56, A61K 31/375, A61K 47/22, A61K 47/10, A61K 47/32

(54) **Arzneimittel auf Basis von Gestagenen zur dermalen Anwendung enthaltend Ascorbinsäure sowie deren Salze**
Medicament based on gestagens for dermal application comprising ascorbic acid and/or salts thereof
Médicament à base de gestagenes à application dermique comprenant l'acide ascorbique et/ou leur sels

(30) Priorität: 21.09.2001 DE 10146541
(43) Veröffentlichungstag der Anmeldung: 26.03.2003
(73) Patentinhaber: Dr. Kade Pharmazeutische Fabrik GmbH, 12277 Berlin (DE)
(72) Erfinder: Franke, Christian, 78476 Allensbach (DE); Beckmann, Karsten, 78333 Stockach (DE)
(74) Vertreter: Kinzebach, Werner

(56) Entgegenhaltungen:
- DE-A- 4 412 464
- PATENT ABSTRACTS OF JAPAN vol. 008, no. 156 (C-234), 19. Juli 1984 (1984-07-19) & JP 59 059612 A (NITTO DENKI KOGYO KK), 5. April 1984 (1984-04-05)
- PATENT ABSTRACTS OF JAPAN vol. 013, no. 227 (C-600), 25. Mai 1989 (1989-05-25) & JP 01 040423 A (KIYUUKIYUU YAKUHIN KOGYO KK;OTHERS: 01), 10. Februar 1989 (1989-02-10)

## Beschreibung

Die vorliegende Erfindung betrifft Arzneimittel auf Basis von Gestagenen zur dermalen Anwendung, beispielsweise in Form eines Gels. Insbesondere werden Kombinationspräparate auf Basis von Norethisteronacetat und Estrogenen, beispielsweise Estradiol, beschrieben. Die Arzneimittel finden Anwendung vor allem im Rahmen der Hormonsubstitutionstherapie bei peri- oder postrnenopausalen Frauen.

Eine nachlassende Estradiolproduktion der Eierstöcke in und nach den Wechseljahren beziehungsweise nach Ovarektomie, führt zu Beschwerden verschiedenster Art. Eine Behandlung dieser Beschwerden erfolgt üblicherweise durch eine Östrogensubstitution, wobei bei einer langfristigen Anwendung östrogenhaltiger Präparate in den Wechseljahren bei nicht hysterektomierten Frauen zusätzlich sequentiell oder kontinuierlich ein Gestagen appliziert werden sollte. Entsprechende Kombinationspräparate sind für die orale Therapie verfügbar (EP-A 0 136 011).

Nasal applizierbare, Progestogen- bzw. Estrogen- oder Gestagen-haltige Arzneimittel sind in der US-A 5,955,454 und DE-A 199 25 290 beschrieben.

Für die transkutane Therapie stehen vor allem wirkstoffhaltige Pflaster zur Verfügung (vgl. z.B. EP-A 0 573 133 und GB-A 2,208,147). Ein Nachteil der Pflaster ist ihre relativ schlechte lokale Verträglichkeit. So kommt es bei einem Teil der Anwenderinnen immer wieder zu sogenannten Pflasterallergien. Hier besitzen halbfeste Zubereitungen wie Gele deutliche Vorteile.

Beispielsweise ist in der EP-A 0 811 381 ein Gel zur transkutanen Applikation von Östrogenen, Gestagenen und Gemischen davon beschrieben. Es wird eine Kombination von Laurylalkohol und Diethylenglykolmonoethylether vorgeschlagen, um eine angemessene transdermale Permeation von Estradiol und Norethisteronacetat zu erzielen. Gemäß WO 90/11064 werden bestimmte Ester, wie Propylenglykolmonolaurat, Methyllaurat und dergleichen, anstatt Laurylalkohol verwendet. Gele zur topischen Anwendung von 19-Norprogestonen sind in der WO 99/48477 beschrieben.

Allerdings stellt sich bei derartigen halbfesten Arzneiformen für einige der üblicherweise verwendeten Sexualhormone und insbesondere für Norethisteron beziehungsweise dessen Derivate sowie weitere Gestagene mit verwandter Struktur die Problematik der oxidativen Wirkstoffzersetzung, die in festen Arzneiformen (z.B. DE-A 44 12 464, die feste Arzneiformen betrifft, welche einen Fettsäureester der Ascorbinsäure zur Stabilisierung von steroidalen Wirkstoffen enthalten können) keine praktische Bedeutung besitzt. Eine Formulierung von halbfesten, Norethisteronhaltigen und den Anforderungen an pharmazeutische Präparate genügenden Zubereitungen lieferte daher im Gegensatz zu festen Präparaten wie Tabletten bisher nur unbefriedigende Ergebnisse.

Die der vorliegenden Erfindung zu Grunde liegende Aufgabe, oxidationsempfindliche Gestagene und vor allem Norethisteron beziehungsweise Norethisteron-Derivate als halbfeste Arzneiform mit der gebotenen Arzneimittelstabilität zu formulieren, löst die vorliegende Erfindung durch den Zusatz von Ascorbinsäure und Ascorbinsäurederivaten. Einem weiteren Aspekt zufolge war es auch Aufgabe, gut verträgliche Formulierungen anzugeben, also beispielsweise solche, die möglichst wenig niedere Alkohole, vor allem wenig Ethanol, enthalten.

Gegenstand der vorliegenden Erfindung sind daher halbfeste, transkutane Arzneimittel auf Basis wenigstens eines oxidationsempfindlichen Gestagens oder eines pharmazeutisch verträglichen Derivats davon, die dadurch gekennzeichnet sind, dass sie Ascorbinsäure und/oder ein pharmazeutisch verträgliches Salz davon enthalten.

Der Anteil erfindungsgemäßer Arzneimittel, der sich auf Ascorbinsäure und deren Salze bezieht, wird auch als Ascorbinsäure-Komponente bezeichnet und kann einzelnen, aber auch einem Gemisch aus zwei oder mehreren verschiedenen Substanzen entsprechen.

Die erfindungsgemäß verwendete Ascorbinsäure beziehungsweise Ascorbinsäuresalze zeichnen sich durch ihre antioxidative und insbesondere stabilisierende Wirkung für sauerstoffempfindliche Stoffe aus und lassen sich in halbfeste Arzneimittel einarbeiten.

Der Begriff "Ascorbinsäure" steht für 5-[1,2-Dihydroxyethyl]-3,4-dihydroxy-5H-furan-2-on der Formel (I)

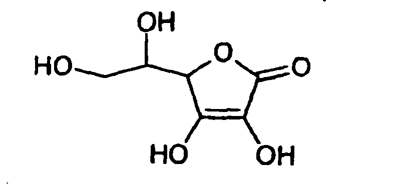

Ferner sind auch Ascorbyl-2-phosphate brauchbar.

Zu den Salzen dieser Verbindungen, d.h. der Ascorbinsäure gehören insbesondere die entsprechenden Basenadditionssalze.

Zu den Basenadditionssalzen zählen Salze mit anorganischen Basen, beispielsweise Metallhydroxiden bzw. -carbonaten von Alkali-, Erdalkali- oder Übergangsmetallen, oder mit organischen Basen, beispielsweise basischen Aminosäuren, wie Arginin und Lysin, Ammoniak, Aminen, z.B. Methylamin, Dimethylamin, Trimethylamin, Triethylamin, Ethylamin, Diethylamin, Ethylendiamin, Ethanolamin, Diethanolamin, Triethanolamin, 1-Amino-2-propanol, 3-Amino-1-propanol oder Hexamethylentetramin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quatemären Ammoniumverbindungen, wie dem Ammonium-lon, Te tramethylammonium-lon und dergleichen.

Bevorzugte Salze werden mit anorganischen Basen gebildet, wie z.B. Na-, K-, Mg-, Ca-, Zn-, Cr- und Fe-Salze, sowie Salze mit dem Ammonium-lon oder quaternären Ammoniumverbindungen.

Bevorzugt sind gut wasserlösliche Formen der Ascorbinsäure, also insbesondere Ascorbinsäure selbst sowie deren Salze.

Die hier gewählte Darstellung der Verbindungen der Formeln (I) und (II) schließt isomere Formen dieser Verbindungen mit ein. Insbesondere zu nennen sind geometrische und Stereoisomere wie cis/trans-Isomere, Enantiomere oder Diastereoisomere, sowie Tautomere, die im vorliegenden Fall vor allem auf die Enolstruktur zurückzuführen sind. Neben den im wesentlichen reinen Isomeren gehören zu den Verbindungen der Formel (I) auch deren isomerengemische, z.B. Stereoisomerengemische. So sind neben den bevorzugten L-isomeren beispielsweise auch Iso-Ascorbinsäure beziehungsweise Iso-AscorbinsäureDerivate zu nennen. Bevorzugt sind die L-Isomeren.

Erfindungsgemäße Arzneimittel enthalten genügend Ascorbinsäure, um die gebotene Arzneimittelstabilität zu gewährleisten. In Bezug auf den Wirkstoffgehalt bedeutet Stabilität im erfindungsgemäßen Sinn eine Abnahme des Gestagen- und insbesondere des Norethisteron-Gehalts von weniger als 10 Gew.-%, und vorzugsweise von weniger als 5 Gew.-%, jeweils bezogen auf die ursprüngliche Wirkstoffmenge, während eines Zeitraums von 36 Monaten bei Raumtemperatur (etwa 25°C). Einem besonderen Aspekt zufolge sollte dabei der Gehalt oxidativer Zersetzungsprodukte wie der Gehalt an 6-Hydroxy- oder 6-Keto-Derivaten, z.B. 6α- und 6β-Hydroxynorethisteron bzw. 6-Ketonorethisteron, oder entsprechender Derivate davon, bei weniger als etwa 2 Gew.-%, vorzugsweise bei weniger als 1 Gew.-% und insbesondere bei weniger als 0,5 Gew.-%, jeweils auf den ursprünglichen Wirkstoffgehalt bezogen, liegen.

In der Regel enthalten erfindungsgemäße Arzneimittel 0,01 bis 1,5 Gew.-%, und vorzugsweise 0,1 bis 1 Gew.-% Ascorbinsäure, beispielsweise etwa 0,2 Gew.-% L-Ascorbinsäure. Ascorbinsäurederivate und -salze werden in entsprechenden Mengen eingesetzt.

In einer besonderen Ausführungsform enthalten erfindungsgemäße Arzneimittel zusätzlich zu Ascorbinsäure, Ascorbinsäuresalzen beziehungsweise Ascorbinsäurederivaten wenigstens ein weiteres Antioxidans.

Diese können insbesondere ausgewählt werden unter Aminosäuren (z. B. Glycin, Histidin, Lysin, Tyrosin, Tryptophan) und deren Derivaten, Imidazolen (z. B. Urocaninsäure) und deren Derivaten, Peptiden wie D,L-Camosin, D-Camosin, L-Camosin und deren Derivaten (z. B. Anserin), Carotinoiden, Carotinen (z. B. α-Carotin, β-Carotin, Lycopin) und deren Derivaten, Chlorogensäure und deren Derivaten, Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl, Butyl- und Lauryl-, Palmitoyl-, Oleyl-, γ-Linoleyl-, Cholesteryl- und Glycerylestem) sowie deren Salzen, Dilaurylthiodipropionat, Distearylthiodipropionat, Thiodipropionsäure und deren Derivaten (Estern, Ethern, Peptiden, Lipiden, Nukleotiden, Nukleosiden und Salzen) sowie Sulfoximinverbindungen (z. B. Buthioninsulfoximinen, Homocysteinsulfoximin, Buthioninsulfonen, Penta-, Hexa, Heptathioninsulfoximin) in sehr geringen verträglichen Dosierungen (z. B. pmol bis mmol/kg), α,β-ungesättigten Carbonsäuren (z.B. Fumarsäure), α-Hydroxysäuren (z. B. Citronensäure, Milchsäure, Apfelsäure), Huminsäure, Gallensäure, Gallenextrakten, Bilirubin, Biliverdin, EDTA, EGTA und deren Derivaten, z.B. Propylgallat, ungesättigten Fettsäuren und deren Derivaten (z. B. γ-Linolensäure, Linolsäure, Ölsäure), Folsäure und deren Derivaten, Ubichinon und Ubichinol und deren Derivaten), Tocopherolen und Derivaten (z. B. Vitamin-E-acetat). Vitamin A und Derivaten (Vitamin-A-palmitat) sowie Koniferylbenzoat des Benzoeharzes, Rutinsäure und deren Derivaten, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Sesamöl, Sesamolin, Stilbenen und deren Derivaten (z. B. Stilbenoxid, Trans-Stilbenoxid) und den erfindungsgemäß geeigneten Derivaten (Salzen, Estern, Ethern, Zuckern, Nukleotiden, Nukleosiden, Peptiden und Lipiden) dieser genannten Antioxidantien.

Erfindungsgemäß bevorzugt ist die Kombination von Ascorbinsäure, Ascorbinsäuresalzen beziehungsweise Ascorbinsäurederivaten mit wenigstens einem Chelatbildner.

Bevorzugte Chelatbildner sind in der Lage, Schwermetallionen zu komplexieren. Insbesondere sind hier Aminopolycarbonsäuren zu nennen, beispielsweise Ethylendiamintetraessigsäure (EDTA), Diethylendiaminpentaessigsäure (DTPA), N-Hydroxyethyldiaminotriessigsäure (HEDTA), Nitrilotriessigsäure beziehungsweise deren Salze. Vorzugsweise wird EDTA oder ein pharmazeutisch verträgliches Salz, beispielsweise das Dinatriumsalz, verwendet.

Soweit vorhanden, enthalten erfindungsgemäße Arzneimittel in der Regel 0,01 bis 1 Gew.-% und vorzugsweise 0,05 bis 0,5 Gew.-% wenigstens eines Chelatbildners, beispielsweise etwa 0,1 Gew.-% EDTA 2Na.

Insbesondere können erfindungsgemäße Arzneimittel auch α-Tocopherol oder α-Tocopherolderivate und/oder vor allem Fumarsäure enthalten. Weitere, in Kombination mit Ascorbinsäure, Ascorbinsäuresalzen bzw. Ascorbinsäurederivaten brauchbare Zusätze sind auch Aminosäuren, vor allem Lysin, und/oder Magnesium- bzw. Aluminiumsulfat.

Der Begriff "α-Tocopherol" bezeichnet erfindungsgemäß 2-[4,8,12-Trimethyltridecyl]-3,4-dihydro-2H-1-benzpyran-6-ol der Formel (III) 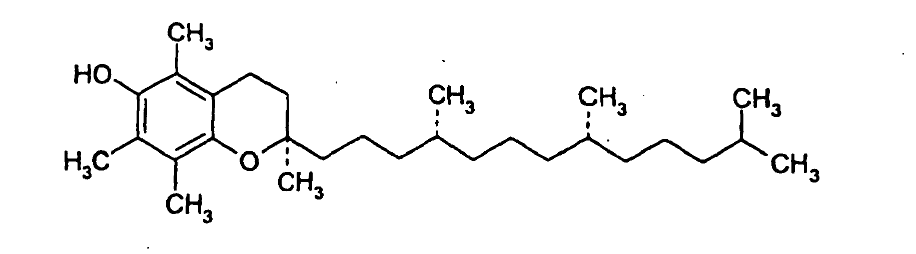 das auch als Vitamin E bezeichnet wird.

Zu den α-Tocopherolderviaten gehören insbesondere α-Tocopherolester, z.B. Ester der Formel (IV) 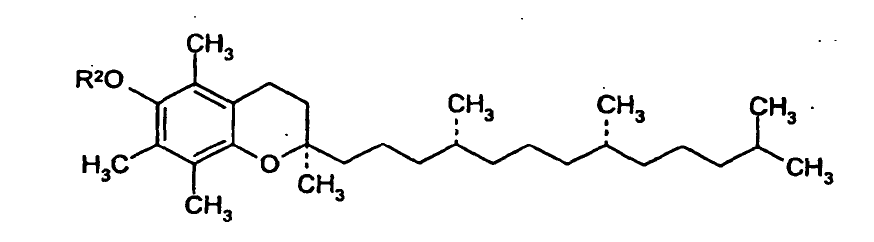 worin R² für einen aliphatischen oder aromatischen Rest mit 1 bis 30 Kohlenstoffatomen steht. α-Tocopherolfettsäureester wie Linol-, Öl-, Linolen,-Palmitin-, Myristin- und Stearinsäureester, α-Tocopherolacetat, α-Tocopherolhydrogensuccinat und α-Tocopherolphosphat sind hier beispielsweise zu nennen.

Soweit vorhanden, enthalten erfindungsgemäße Arzneimittel in der Regel 0,01 bis 1,5 Gew.-% und vorzugsweise 0,05 bis 1 Gew.-% wenigstens eines weiteren Antioxidans, insbesondere etwa 0,1 Gew.-% Lysin und/oder Fumarsäure.

Zu den oxidationsempfindlichen Gestagenen gehören vor allem Gestagene mit einem in 4,5-Position ungesättigten Steroid-Grundgerüst der Formel (V) 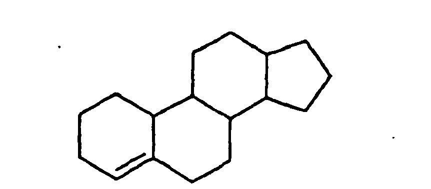 Insbesondere sind dies

Allylestrenol (17α-Allyl-4-estren-17-ol) der Formel (Va)

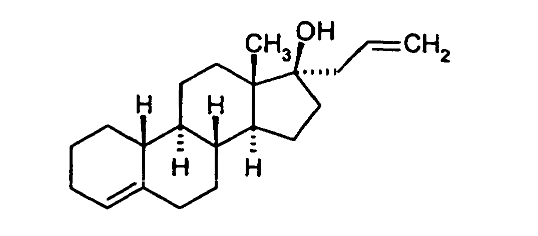

Desogestrel (13-Ethyl-11-methylen-18,19-dinor 17α-pregn-4-en-20-in-17-ol) der Formel (Vb)

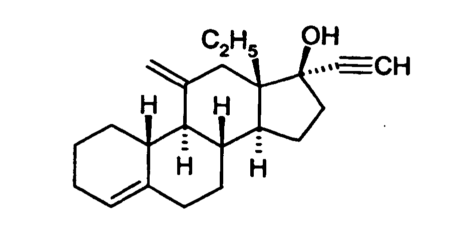

Gestoden (13-Ethyl-17β-hydroxy-18,19-dinor-4,15-pregnadien-20-in-3-on) der Formel (Vc)

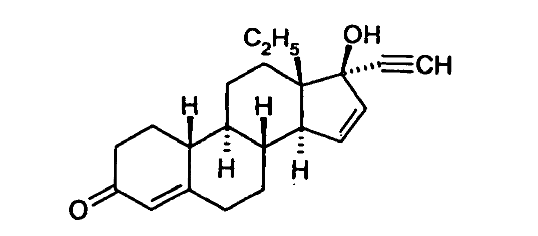

Hydroxyprogesteron (17α-Hydroxypregn-4-en-3,20-dion) der Formel (Vd)

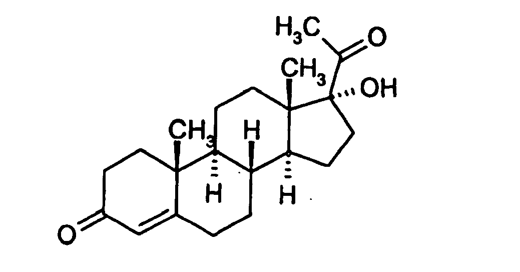

Lynestrenol (19-Nor-17α-pregn-4-en-20-in-17-ol) der Formel (Ve)

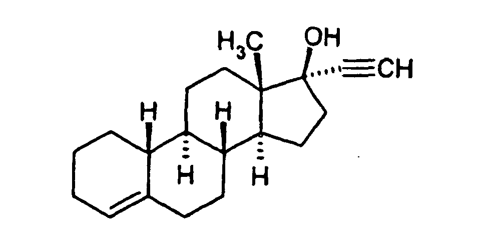

Norgestrel ((±)-13-Ethyl-17-hydroxy-18,19-dinor-17α-pregn-4-en-20-in-3-on) und Levonorgestrel ((-)-13-Ethyl-17-hydroxy-18,19-dinor-17α-pregn-4-en-20-in-3-on) der Formel (Vf)

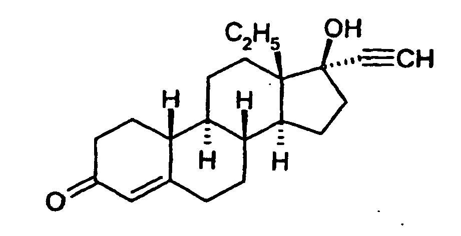

Erfindungsgemäß bevorzugt ist Norethisteron (17-Hydroxy-19-nor-17α-pregn-4-en-20-in-3-on) der Formel (Vg) 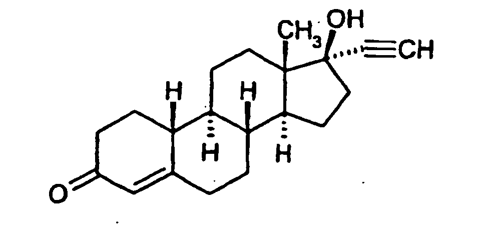 das auch als 17α-Ethinyl-19-nortestosteron, Norethindron oder Norpregneminolon bezeichnet wird.

Zu den erfindungsgemäß verwendbaren Gestagenderivaten gehören insbesondere deren Ester, die transkutan appliziert werden können. Hierzu zählen vor allem Acetate, Enanthate, Caproate, Valerate und weitere pharmazeutisch verträgliche Ester mit C₂-C₁₀-Alkanoylresten, die vornehmliich an die Hydroxygruppe in Position 17 gebunden sind. Norethisteronacetat, d.h. 17β-Hydroxy-19-nor-17α-pregn-4-en-20-in-3-onacetat der Formel (Vg1) 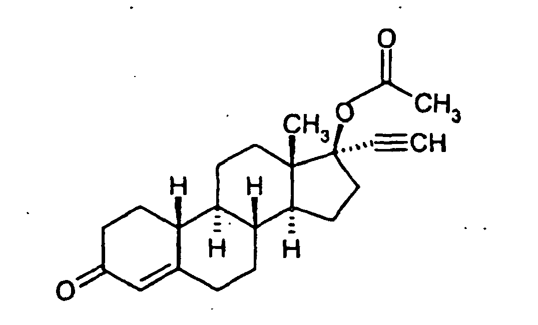 das auch als 17α-Ethinyl-19-nor-testosteronacetat bezeichnet wird, ist besonders bevorzugt.

Als ein weiterer Norethisteronester wäre Norethisteronenanthat zu nennen.

Erfindungsgemäße Arzneimittel enthalten eine wirksame Menge an Norethisteron. In Abhängigkeit von der gewünschten Dosierung beträgt der Gehalt an Norethisteron in der Regel 0,01 bis 1,5 Gew.-% und vorzugsweise 0,1 bis 0,5 Gew.-%. Norethisteronderivate werden in entsprechenden Mengen zugesetzt, beispielsweise etwa 0,186 Gew.-% Norethisteronacetat (entsprechend 0.163 Gew.-% Norethisteron).

Eine besonders vorteilhafte Ausführungsform der vorliegenden Erfindung betrifft Arzneimittel, in denen das Gestagen, insbesondere Norethisteronacetat, in gelöster Form vorliegt.

Gemäß einer weiteren besonderen Ausführungsform betrifft die vorliegende Erfindung Arzneimittel mit einer Wirkstoffkombination, die neben Norethisteron beziehungsweise Norethisteronderivaten weitere, insbesondere mit Gestagenen zu kombinierende Wirkstoffe enthalten.

Gemäß einer bevorzugten Ausführungsform erhalten erfindungsgemäße Arzneimittel neben Norethisteron beziehungsweise Norethisteronderviaten wenigstens ein transkutan verabreichbares Estrogen, von denen vor allem Estradiol und Estradiolderivate zu nennen sind.

Der Begriff Estradiol bezeichnet erfindungsgemäß 3,17β-Dihydroxy-Δ^{1,2,5,(10)-}Estratrien der Formel (VI)

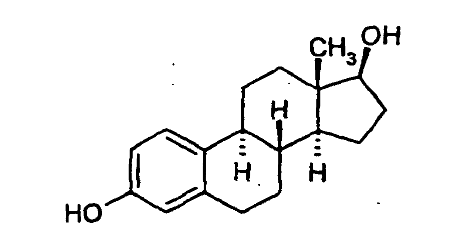

Estradiol wird gewöhnlich als Anhydrat oder als Hemihydrat eingesetzt.

Zu erfindungsgemäß verwendbaren Estradiolderivaten gehören insbesondere Estradiolester, z.B. Ester der Formel (VIII) 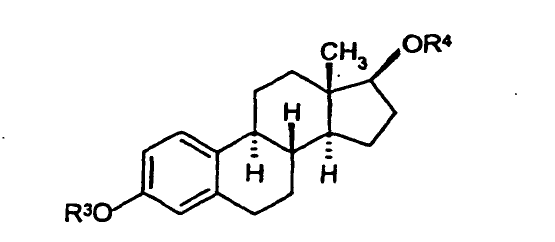 worin R³ und R⁴ unabhängig voneinander für einen aliphatischen oder aromatischen Rest mit 1 bis 30 Kohlenstoffatomen stehen. Estradiol-3-benzoat, Estradiol-17-cypionat, Estradiol-3,17-dipropionat, Estradiol-17-undecylat und Estradiol-17-valerat sind hier beispielsweise zu nennen.

Soweit vorhanden, enthalten erfindungsgemäße Arzneimittel eine in Kombination mit Norethisteron wirksame Menge wenigstens eines Estrogens. In der Regel beträgt der Gehalt an Estrogen 0,01 bis 1,5 Gew.-% und vorzugsweise 0,02 bis 0,5 Gew.-%, beispielsweise etwa 0,06 Gew.-% Estradiol. Estradiolderivate werden in entsprechenden Mengen verwendet.

Eine besonders bevorzugte Ausführungsform betrifft ein erfindungsgemäßes Arzneimittel auf Basis von Norethisteronacetat und Estradiol.

Die erfindungsgemäßen Arzneimittel gehören in den Bereich der halbfesten Arzneiformen. Halbfest im erfindungsgemäßen Sinne besitzt hierbei die allgemein übliche in den einschlägigen Arzneibüchern, Monographien und Pharmakopöen angegebene Bedeutung. Es handelt sich insbesondere um streichfähige Zubereitungen, deren streichfähige Konsistenz bei Raumtemperatur, das heißt in der Regel etwa 20 bis 25°C, gegeben sein sollte.

Erfindungsgemäße streichfähige Dermatika können dermatologischen Aspekten zufolge in Salben, Cremes, Gele und Pasten eingeteilt werden, wobei aus pharmazeutischer Sicht der Begriff "Salbe" für alle streichfähigen Zubereitungen zur dermalen Anwendung, d.h. auf Haut oder Schleimhaut, verwendet werden kann.

Die erfindungsgemäßen halbfesten Arzneiformen enthalten eine einfache oder zusammengesetzte Grundlage, in der Norethisteron beziehungsweise Noresthisteronderivate gelöst oder dispergiert sind. Die Grundlagen können aus natürlichen oder synthetischen Substanzen gebildet werden, sie können Ein- oder Mehrphasensysteme sein und je nach Art der Grundlage hydrophile oder hydrophobe (lipophile) Eigenschaften aufweisen.

Salben sind erfindungsgemäße halbfeste Arzneiformen mit einer einheitlichen Grundlage, in welcher feste oder flüssige Substanzen gelöst und/oder dispergiert sein können.

Cremes sind erfindungsgemäße mehrphasige Zubereitungen, die aus einer lipophilen und einer wässrigen Phase bestehen.

Gele basieren auf gelierten Flüssigkeiten, die mit Hilfe geeigneter Quellmittel (Gelbildner) erhältlich sind.

Pasten sind erfindungsgemäße halbfeste Zubereitungen, deren Grundlagen erhebliche Anteile fein dispergierter Pulver enthalten.

Geeignete Grundlagen lassen sich beispielsweise mit Kohlenwasserstoffen, insbesondere Vaseline, Triglyceriden, zum Beispiel flüssigen Fetten, wie Erdnussöl, Olivenöl, Sonnenblumenöl und Rizinusöl, und streichfähigen Fetten wie Schweinefett, neben diesen natürlichen Fetten auch hydrierten Fetten wie hydriertem Rizinusöl, und synthetischen Fetten; Polyethylenglycolen (Macrogolen) mit geringen bis mittleren Molekulargewichten, die sich in der Regel im Bereich von 200 bis 5000 bewegen, beispielsweise Polyethylenglycol 300, 400, 1500 oder 4000 und insbesondere Gemischen davon; wasseraufnehmenden Grundlagen, auch als Absorbtionsbasen bezeichnet, beispielsweise lipophilen wasseraufnehmenden Grundlagen wie Wollwachs, und hydrophilen wasseraufnehmenden Grundlagen wie emulgierendem Cetylstearylalkohol; Gelbildnem, beispielsweise anorganischen Hydrogelbildnem oder organischen Hydrogelbildnem; Wasser und weiteren hydrophilen Lösungsmitteln, wie kurzkettigen Alkoholen, z.B. Ethanol, formulieren.

Gemäß einer bevorzugten Ausführungsform sind erfindungsgemäße halbfeste Arzneimittel als Gel und insbesondere als Hydrogel formuliert.

Hierbei handelt es sich um streichfähige Zubereitungen mit einer insbesondere hydrophilen Phase.

Die hydrophile Phase wird in der Regel aus Lösungsmittel, also insbesondere aus Wasser und gegebenenfalls hydrophilen Lösungsmitteln, beispielsweise kurzkettigen Alkoholen wie Ethanol, oder Polyethylenglycolen mit niedrigem bis mittlerem Molekulargewicht gebildet. Der Anteil an hydrophiler Phase beträgt in der Regel 50 bis 99 Gew.-% und vorzugsweise 70 bis 95 Gew.-%, beispielsweise etwa 50 Gew.-% Wasser und 40 Gew.-% Ethanol. Vorzugsweise enthält das Arzneimittel möglichst wenig kurzkettige Alkohole, insbesondere Ethanol. Ein Gehalt von weniger als 50 Gew.-% ist von Vorteil.

Als Gelbildner eignen sich zum Beispiel:
a) anorganische Gelbildner, wie
   - hochdisperses Siliciumdioxid sowie dessen Mischungen mit Aluminiumoxid;
   - Magnesium-Aluminiumsilikate z.B. Bentonite;
b) organische Gelbildner, wie:
   - native Substanzen, insbesondere Gelatine, Polysaccharide (z.B. Stärke, Pektin, Tragant, Alginate, Xantangummi);
   - halbsynthetische Gelbildner, insbesondere Celluloseether (z.B. Methylcellulose, Ethylcellulose, Hydroxyethylcellulose, Hydroxypropylcellulose, Hydroxypropylmethylcellulose, Natriumcarboxymethylcellulose), Stärkederivate, Pektinderivate;
   - vollsynthetische Gelbildner wie Polyvinylalkohol, Polyvinylpyrrolidone, Ethylenoxid-Propylenoxid-Blockcopolymere, Carboxyvinylpolymere sowie Polymere und Copolymere der Acryl- beziehungsweise Methacrylsäure.

Erfindungsgemäß bevorzugt werden die üblicherweise als Gelbildner verwendeten Acrylsäure-Polymere-(CTFA-Bezeichnung: Carbomer) oder die zuvor genannten Cellulosederivate.

Die Menge an Gelbildner wird üblicherweise so bemessen, dass sich die gewünschten rheologischen Eigenschaften des resultierenden Gels einstellen. Demnach können Gele eine elastische Beschaffenheit (Gallerte) aufweisen oder plastisch verformbar sein. In der Regel werden 0,1 bis 10 Gew.-% und vorzugsweise 0,5 bis 5 Gew.-% Gelbildner, beispielsweise etwa 1,4 Gew.-% Carbomer verwendet.

Ferner enthalten die erfindungsgemäßen halbfesten Arzneimittel und insbesondere die Gele neben Lösungsmittel vorteilhafterweise auch einen oder mehrere Lösungsvermittler. Eine Funktion geeigneter Lösungsvermittler kann die Förderung der perkutanen Resorption der Wirkstoffe sein. Brauchbare Lösungsvermittler können daher aus der Gruppe der Permeationsbeschleuniger ausgewählt werden. Zu nennen sind beispielsweise ein- oder mehrwertige Alkohole wie Benzylalkohol, Ethylenglycol, Propylenglycol, Glycerol und Sorbitol, sowie deren Ether und Ester, beispielsweise Diethylenglycolmonoalkylether mit vorzugsweise 1 bis 4 Kohlenstoffatomen im Alkylteil, insbesondere Diethylenglycolmonoethylether, Dimethylisosorbit oder Glycerylcaprylat. Ebenso geeignet sind ethoxylierte Glyceride wie z.B. PEG-6-Capryl/Caprinsäure-Glyceride. Auch Emulgatoren können geeignet sein, wie z.B. Polysorbate, Sorbitanfettsäureester oder Polyethylenglycol-400-stearat. Bei Gebrauch dieser Lösungsvermittler kann der Anteil kurzkettiger Alkohole verhältnismäßig gering gewählt werden.

Soweit vorhanden, enthalten erfindungsgemäße Arzneimittel in der Regel 0,5 bis 20 Gew.-% und vorzugsweise 1 bis 10 Gew.-% Lösungsvermittler, beispielsweise etwa 5 Gew.-% Diethylenglycolmonoethylether.

Darüber hinaus können die erfindungsgemäßen halbfesten Arzneimittel weitere geeignete Zusätze wie Neutralisationsmittel, beispielsweise Triethanolamin, Natronlauge, Tris-Puffer, Konservierungsmittel; Hautöle; Hautschutzstoffe; Hautpflegemittel enthalten.

Gemäß einer bevorzugten Ausführungsform der vorliegenden Erfindung enthalten die Arzneimittel
- 0,01 bis 1,5 Gew.-% und insbesondere etwa 0,186 Gew.-% Norethisteronacetat oder eine bioäquivalente Menge eines Norethisteronderivates;
- 0,01 bis 1,5 Gew.-% und insbesondere etwa 0,06 Gew.-% Estradiol oder eine bioäquivalente Menge eines Estradiol;
- 0,01 bis 1,5 Gew.-% und insbesondere etwa 0,2 Gew.-% Ascorbinsäure oder eine in Bezug auf die Redoxwirkung entsprechende Menge eines Salzes davon.

Im Rahmen der vorstehend beschriebenen Ausführungsform können die Arzneimittel vorteilhafterweise:
- 0,01 bis 1,0 Gew.-% und insbesondere etwa 0,1 Gew.-% EDTA oder eine in Bezug auf die Chelatbildung entsprechende Menge anderer Chelatbildner und/oder
- 0,01 bis 1,5 Gew.-% und insbesondere etwa 0,1 Gew.-% Lysin und/oder Fumarsäure oder eine in Bezug auf die Redoxwirkung entsprechende Menge an Salzen davon
enthalten.

Im Rahmen der vorstehend beschriebenen Ausführungsform enthalten Hydrogele:
- 0,1 bis 10 Gew.-% und insbesondere etwa 1,4 Gew.-% Carbomer oder eine in Bezug auf die Gelbildung entsprechende Menge anderer Gelbildner, und
- 50 bis 99 Gew.-% und insbesondere etwa 90 Gew.-% eines wässrigethanolischen Gemisches oder eine entsprechende Menge eines hydrophilen Lösungsmittels oder Lösungsmittelgemisches.

Vorteilhafterweise können die im Rahmen der vorstehend beschriebenen Ausführungsform genannten Hydrogele:
- 0,5 bis 20 Gew.-% und insbesondere etwa 5 Gew.-% Diethylenglykolmonoethylether oder eine in Bezug auf die solubilisierende Wirkung entsprechende Menge anderer Lösungsvermittler; und/oder
- 0,1 bis 5 Gew.-% und insbesondere etwa 1,6 Gew.-% Triethanolamin oder eine in Bezug auf die Basizität entsprechende Menge anderer Neutralisationsmittel;
enthalten, wobei der Ethanol-Anteil vorteilhafterweise weniger als 50 Gew.-% und insbesondere weniger als 45 Gew.-% betragen kann.

Die Herstellung der Arzneimittel erfolgt in an sich bekannter Weise. Die dazu erforderlichen Ausgangstoffe sind literaturbekannt.

Die Verabreichung eines erfindungsgemäßen Arzneimittels erfolgt in der Regel durch ein- oder mehrmaliges Auftragen und Einreiben einer der gewünschten Dosierung entsprechenden Menge an halbfester Zubereitung auf die Haut oder Schleimhaut, beispielsweise der Arme, der Oberschenkel oder des Unterleibs. Üblicherweise ist die Wirkstoffkonzentration in der Zubereitung so bemessen, dass etwa 0,5 bis 5 g Gel täglich aufzutragen sind.

Erfindungsgemäße Arzneimittel werden insbesondere im Bereich der Hormonsubstitutionstherapie verwendet, also insbesondere zur Behandlung von Beschwerden bei nachlassender Estradiolproduktion der Eierstöcke in und nach den Wechseljahren beziehungsweise nach Ovarektomie (klimakterisches Syndrom, zum Beispiel Hitzewallungen, nächtliches Schwitzen, atrophische Erscheinungen im Urogenitaltrakt). Die Anwendung kann im Rahmen einer sequentiellen oder kontinuierlichen Therapie erfolgen.

Angaben in Gew.-% beziehen sich, soweit nicht anders angegeben, auf ein Gesamtgewicht des Arzneimittels (der Formulierung).

Die vorliegende Erfindung wird nun an Hand des folgenden Beispiels erläutert:

### Beispiel 1: Estradiol/Norethisteronacetat-Gel

Folgende Komponenten wurden in an sich bekannter Weise zu einem Gel verarbeitet:

| Komponente | Menge [g] |
|---|---|
| Estradiol 1/2 H₂O | 0,62 |
| Norethisteronacetat | 1,86 |
| EDTA 2Na | 1,00 |
| Ascorbinsäure | 2,00 |
| Carbomer | 14,00 |
| Triethanolamin | 16,00 |
| Diethylenglycolmonoethylether | 50,00 |
| Ethanol 96% | 417,00 |
| gereinigtes Wasser | 497,52 |
| | 1000,00 |

### Beispiel 2: Stabilitätsvergleich

Ein mit Beispiel 1 vergleichbares, zusätzlich aber α-Tocopherol enthaltendes Gel A wurde einem Stabilitätstest (ca. 22 Monate bei Raumtemperatur) unterzogen. Es wurde das Ausmaß der Wirkstoffzersetzung bestimmt und verglichen mit einem entsprechenden Gel B, das allerdings keine Ascorbinsäure, wohl aber α-Tocopherol enthielt und etwa 18 Monate bei Raumtemperatur gelagert worden war:

| Zersetzungsprodukte | Gel A | Gel B |
|---|---|---|
| 6α-, 6β-Hydroxynorethisteronacetat | 0,24% | 1,83% |
| 6-Ketonorethisteronacetat | 0,05% | 1,71% |
| Gesamt | 0,90% | 6,37% |

Es ist klar ersichtlich, dass das erfindungsgemäße Gel eine wesentlich bessere Wirkstoffstabilität besaß als das vergleichbare Gel, das lediglich α-Tocopherol als Antioxidans, aber keine Ascorbinsäure enthielt:

## Patentansprüche

1. Halbfestes, transkutanes Arzneimittel auf Basis wenigstens eines oxidationsempfindlichen Gestagens oder eines pharmazeutisch verträglichen Derivats davon, **dadurch gekennzeichnet, daß** es Ascorbinsäure und/oder ein pharmazeutisch verträgliches Salz davon enthält.

2. Arzneimittel nach Anspruch 1, **dadurch gekennzeichnet, daß** der Gehalt an Ascorbinsäure 0,01 bis 1,5 Gew.-% beträgt.

3. Arzneimittel nach Anspruch 1 oder 2, **dadurch gekennzeichnet, daß** es wenigstens einen Chelatbildner enthält.

4. Arzneimittel nach Anspruch 3, **dadurch gekennzeichnet, daß** der Chelatbildner EDTA oder ein pharmazeutisch verträgliches Salz davon ist.

5. Arzneimittel nach Anspruch 3, **dadurch gekennzeichnet, daß** der Gehalt an Chelatbildner 0,01 bis 1 Gew.-% beträgt.

6. Arzneimittel nach einem der vorhergehenden Ansprüche, **dadurch gekennzeichnet, daß** es wenigstens einen Lösungsvermittler enthält.

7. Arzneimittel nach Anspruch 6, **dadurch gekennzeichnet, daß** der Lösungsvermittler ein Diethylenglykolmono-C₁₋₄-alkylether oder Dimethylisosorbit ist.

8. Arzneimittel nach Anspruch 6 oder 7, **dadurch gekennzeichnet, daß** der Ethanol-Gehalt des Arzneimittels weniger als 50 Gew.-% beträgt.

9. Arzneimittel nach einem der vorhergehenden Ansprüche, **dadurch gekennzeichnet, daß** das Gestagen eine in Position 4,5 einfach ungesättigte Steroid-Grundstruktur aufweist

10. Arzneimittel nach Anspruch 9, **dadurch gekennzeichnet, daß** das Gestagen Allylestrenoll, Desogestrel, Gestoden, Hydroxyprogesteron, Lynestrenol, Norethisteron, Norgestrel, Levonorgestrel oder ein pharmazeutisch verträgliches Derivat davon ist.

11. Arzneimittel nach Anspruch 10, auf Basis von Norethisteronacetat.

12. Arzneimittel nach Anspruch 11, **dadurch gekennzeichnet, daß** der Gehalt an Norethisteron 0,01 bis 1,5 Gew.-% beträgt.

13. Arzneimittel nach einem der vorhergehenden Ansprüche, **dadurch gekennzeichnet, daß** es ein transkutan verabreichbares Estrogen enthält.

14. Arzneimittel nach Anspruch 13, **dadurch gekennzeichnet, daß** das transkutan verabreichbare Estrogen Estradiol oder ein pharmazeutisch verträgliches Derivat davon ist.

15. Arzneimittel nach Anspruch 13, **dadurch gekennzeichnet, daß** der Gehalt an Estrogen 0,01 bis 1,5 Gew.-% beträgt.

16. Arzneimittel nach einem der vorhergehenden Ansprüche, in Form eines Gels.

## Claims

1. A semisolid transcutaneous medicament based on at least one oxidation-sensitive progestin or a pharmaceutically acceptable derivative thereof, which comprises ascorbic acid and/or a pharmaceutically acceptable salt thereof.

2. A medicament as claimed in claim 1, wherein the ascorbic acid content is from 0.01 to 1.5% by weight.

3. A medicament as claimed in claim 1 or 2, which comprises at least one chelating agent.

4. A medicament as claimed in claim 3, wherein the chelating agent is EDTA or a pharmaceutically acceptable salt thereof.

5. A medicament as claimed in claim 3, wherein the chelating agent content is from 0.01 to 1% by weight.

6. A medicament as claimed in claim 1, which comprises at least one solubilizer.

7. A medicament as claimed in claim 6, wherein the solubilizer is a diethylene glycol mono-C₁₋₄-alkyl ether or dimethylisosorbitol.

8. A medicament as claimed in claim 6 or 7, wherein the ethanol content of the medicament is less than 50% by weight.

9. A medicament as claimed in any one of the preceding claims, wherein the progestin has a basic steroid structure which is monounsaturated in the 4,5 position.

10. A medicament as claimed in claim 9, wherein the progestin is allylestrenol, desogestrel, gestodene, hydroxyprogesterone, lynestrenol, norethisterone, norgestrel, levonorgestrel or a pharmaceutically acceptable derivative thereof.

11. A medicament as claimed in claim 10, based on norethisterone acetate.

12. A medicament as claimed in claim 11, wherein the norethisterone content is from 0.01 to 1.5% by weight.

13. A medicament as claimed in any one of the preceding claims, which comprises an estrogen which can be administered transcutaneously.

14. A medicament as claimed in claim 13, wherein the estrogen which can be administered transcutaneously is estradiol or a pharmaceutically acceptable derivative thereof.

15. A medicament as claimed in claim 13, wherein the estrogen content is from 0.01 to 1.5% by weight.

16. A medicament as claimed in any one of the preceding claims in the form of a gel.

## Revendications

1. Médicament transcutané semi-solide à base d'au moins un gestagène sensible à l'oxydation ou d'un dérivé pharmaceutiquement acceptable de celui-ci, **caractérisé en ce qu'**il contient de l'acide ascorbique et/ou un sel pharmaceutiquement acceptable de celui-ci.

2. Médicament selon la revendication 1, **caractérisé en ce que** la teneur en acide ascorbique va de 0,01 à 1,5 % en poids.

3. Médicament selon la revendication 1 ou 2, **caractérisé en ce qu'**il contient au moins un chélateur.

4. Médicament selon la revendication 3, **caractérisé en ce que** le chélateur est l'EDTA ou un sel pharmaceutiquement acceptable de celui-ci.

5. Médicament selon la revendication 3, **caractérisé en ce que** la teneur en chélateur va de 0,01 à 1 % en poids.

6. Médicament selon l'une des revendications précédentes, **caractérisé en ce qu'**il contient au moins un solubilisant.

7. Médicament selon la revendication 6, **caractérisé en ce que** le solubilisant est un monoalkyl(C₁-C₄)éther de diéthylèneglycol ou le diméthyl-isosorbitol.

8. Médicament selon la revendication 6 ou 7, **caractérisé en ce que** la teneur en éthanol du médicament est inférieure à 50 % en poids.

9. Médicament selon l'une des revendications précédentes, **caractérisé en ce que** le gestagène présente une structure de base stéroïdienne insaturée en position 4,5.

10. Médicament selon la revendication 9, **caractérisé en ce que** le gestagène est l'allylestrénol, le désogestrel, le gestodène, l'hydroxyprogestérone, le lynestrénol, la noréthistérone, le norgestrel, le lévonorgestrel ou un dérivé pharmaceutiquement acceptable de ceux-ci.

11. Médicament selon la revendication 10, à base d'acétate de noréthistérone.

12. Médicament selon la revendication 11, **caractérisé en ce que** la teneur en noréthistérone va de 0,01 à 1,5 % en poids.

13. Médicament selon l'une des revendications précédentes, **caractérisé en ce qu'**il contient un oestrogène administrable par voie sous-cutanée.

14. Médicament selon la revendication 13, **caractérisé en ce que** l'oestrogène administrable par voie transcutanée est l'oestradiol ou un de ses dérivés pharmaceutiquement acceptable.

15. Médicament selon la revendication 13, **caractérisé en ce que** la teneur en oestrogène est de 0,01 à 1,5 % en poids.

16. Médicament selon l'une quelconque des revendications précédentes, sous forme d'un gel.
